# EUROPÄISCHE PATENTSCHRIFT

(11) **EP 1 204 426 B1**
(45) Veröffentlichungstag und Bekanntmachung des Hinweises auf die Patenterteilung: **24.08.2005**
(21) Anmeldenummer: 00967511.7
(22) Anmeldetag: 09.08.2000
(51) Int. Cl.: A61K 41/00, A61K 51/12, A61P 19/02

(54) **MEDIZINISCHES PRÄPARAT ZUR BEHANDLUNG VON ARTHROSE, ARTHRITIS UND ANDEREN RHEUMATISCHEN GELENKERKRANKUNGEN**
MEDICAL PREPARATION FOR TREATING ARTHROSIS, ARTHRITIS AND OTHER RHEUMATIC JOINT DISEASES
PREPARATION MEDICALE POUR TRAITER L'ARTHROSE, ARTHRITE ET D'AUTRES AFFECTIONS ARTICULAIRES RHUMATISMALES

(30) Priorität: 19.08.1999 DE 19940220
(43) Veröffentlichungstag der Anmeldung: 15.05.2002
(73) Patentinhaber: Magforce Applications GmbH, 14050 Berlin (DE)
(72) Erfinder: JORDAN, Andreas, D-14167 Berlin (DE)
(74) Vertreter: Arth, Hans-Lothar
(86) Internationale Anmeldenummer: PCT/DE2000/002720
(87) Internationale Veröffentlichungsnummer: WO 2001/013949

(56) Entgegenhaltungen:
- DE-A- 19 726 282
- US-A- 4 758 429
- US-A- 5 149 319
- GRADY E.D. ET AL: "Combination of internal radiation therapy and hyperthermia to treat liver cancer." SOUTHERN MEDICAL JOURNAL, (1983) 76/9 (1101-1105). CODEN: SMJOAV, XP001015654
- DATABASE BIOSIS [Online] BIOSCIENCES INFORMATION SERVICE, PHILADELPHIA, PA, US; MASAI, AKIRA: "Development of Anticancer-Agent-Releasing Microcapsules for Chemotherapy Combined with Embolo- Hyperthermic Therapy." retrieved from STN Database accession no. PREV199598223481 XP002174710 & NIPPON ACTA RADIOLOGICA, (1995) VOL. 55, NO. 1, PP. 50-57.,

## Beschreibung

Die Erfindung betrifft die Verwendung einer Suspension aus nanoskaligen magnetischen Teilchen zur Herstellung eines medizinischen Präparats zur Behandlung von Arthrose, Arthritis und anderen rheumatischen Gelenkerkrankungen.

Zur Behandlung von chronisch schmerzhaften und mit dauerhaften Bewegungseinschränkungen verbundenen Störungen des Stütz- und Bewegungsapparates, wie Arthritis, Arthrose und rheumatischen Erkrankungen, wird neben physikalischen Therapieformen in erster Linie eine systemische Pharmakotherapie mit Analgetika und nichtsteroidalen Antiphlogistika sowie Kortison angewendet, die zum einen die Ursachen der Erkrankung nicht bekämpft und zudem mit unerwünschten Nebenwirkungen verbunden ist.

Bei einer anderen bekannten Therapieform, der Radiosynoviorthese, werden zur unmittelbaren Bekämpfung der krankhaften Wucherung der Gelenkinnenhaut stabilisierte Isotope mit einer Reichweite von nur wenigen Millimetern in das betreffende Gelenk gespritzt, um durch Strahlung die Zellen der Gelenkinnenhaut zu schädigen und den Entzündungsprozeß dadurch einzudämmen.

Die Wirksamkeit dieser sogenannten Radiosynoviorthese setzt jedoch die Phagozytoseaktivität der in der Gelenkflüssigkeit aufgrund der Entzündung vermehrt vorhandenen Makrophagen voraus, die jedoch mit zunehmendem Alter der Patienten abnimmt, so daß der erfolgreichen Anwendung der Radiosynoviorthese Grenzen gesetzt sind.

Gemäß der US 47 58 429 werden dem erkrankten Patienten intravenös kolloidale Suspensionen aus magnetischen, paramagnetischen oder diamagnetischen Teilchen in Form von Fe₂O₃ oder FeOOH in der Größe von weniger als 1 Mikrometer gegeben, die mit Beschichtungen aus Dextran, Metalloporphyrinen sowie anorganischen und organischen Metallverbindungen als Substituenten im Eisenkern versehen sind. Das Anlegen eines magnetischen Wechselfeldes soll in der erkrankten Region eine Energiezufuhr bewirken, wobei unklar ist, wie die beschichteten Eisenoxidteilchen zu der erkrankten Stelle gelangen sollen. Kolloidale Suspensionen auf der Basis von derart beschichteten Eisenoxidteilchen sind nicht in der Lage, die entzündlichen Vorgänge bei Arthritis, Arthrose und anderen rheumatischen Gelenkerkrankungen entscheidend zurückzudrängen.

Aus dem Bereich der Tumortherapie durch Hyperthermie sind durch die DE 197 26 282 bereits nanoskalige Teilchen mit einem eisenoxidhaltigen ferri-, ferro- oder superparamagnetischen Kern und mindestens zwei den Kern umgebenden Schalen bekannt, wobei die dem Kern benachbarte Schale viele positiv geladene funktionelle Gruppen aufweist, die eine leichte Aufnahme des so umhüllten eisenoxidhaltigen Kerns in das Innere der Tumorzelle ermöglichen, wobei diese Schale vom Tumorgewebe so langsam abgebaut wird, daß der Kern ausreichend Zeit hat, sich an die Zelloberfläche anzuheften, um anschließend in das Zellinnere aufgenommen zu werden. Die äußeren Schalen werden von Spezies aufgebaut, die die darunterliegenden positiv geladenen Gruppen der inneren Schale so abschirmen, daß die nanoskaligen Teilchen nach außen hin insgesamt neutral oder negativ geladen erscheinen. Die in Form eines magnetischen Fluids punktförmig in das Tumorgewebe injizierten Teilchen werden aufgrund ihrer oben beschriebenen Ausbildung zum einen gut im Tumorgewebe verteilt und zum anderen auch gut in das Innere der Tumorzellen eingeschleust. Nach Anlegen eines magnetischen Wechselfeldes und der dabei entstehenden Temperaturen ist aufgrund der guten Verteilung der nanoskaligen Teilchen eine erfolgreiche Tumortherapie durch Hyperthermie möglich.

Aus dem Aufsatz von GRADY, E.D. et al: Combination of internal radiation therapy and hyperthermia to treat liver cancer" in Southern Medical Journal (1983) 76/9 (1101-1105) ist ein Verfahren zur Behandlung von Leberkrebs in einer Kombination aus innerer Strahlungsbehandlung mit intraarteriellen Yttrium-90-Teilchen sowie regionaler Hyperthermie mit elektromagnetischer Strahlung bekannt.

Masai, Akira: "Development of Anticancer-Agent-Releasing Microcapsules for Chemotherapy Combined with Embolo-Hyperthermic Therapy" Dep. Radiol., Fukushima Med. Coll., Fukushima, Japan Nippon Acta Radiologica (1995) Vol. 55, No.1, PP 50-57 beschreibt die Anwendung von aus ferromagnetischen, amorphem Metall hergestellten Microkapseln, die außerdem in der Lage sind, ein Anti-Krebs-Präparat freizusetzen. Die Kapseln erzeugen in einem magnetischen Wechselfeld Wärme zur Kombination von Hyperthermie und Chemotherapie bei der Krebsbehandlung.

Die bekannten nanoskaligen Teilchen sind für die Behandlung von Gelenkerkrankungen nicht vorgesehen und in der vorliegenden Form auch nicht erfolgverbürgend einsetzbar.

Der Erfindung liegt daher die Aufgabe zugrunde, ein Mittel zur lokalen und systemischen Behandlung von Arthrose, Arthritis und anderen rheumatischen Gelenkerkrankungen anzugeben, mit dem auf der Grundlage der von in einem magnetischen Wechselfeld befindlichen Eisenoxidteilchen gelieferten Wärme eine erfolgreiche Schmerzbekämpfung und Eindämmung der Entzündungsprozesse in den betroffenen Gelenken gewährleistet ist.

Erfindungsgemäß wird die Aufgabe durch eine entsprechend den Merkmalen des Patentanspruches 1 gekennzeichnete Verwendung von nanoskaligen Teilchen zur Herstellung eines Präparates zur Behandlung von Gelenkerkrankungen gelöst.

Aus den abhängigen Ansprüchen sowie der unten vorgenommenen Beschreibung eines Ausführungsbeispiels ergeben sich weitere Merkmale und vorteilhafte Weiterbildungen der Erfindung.

Der Grundgedanke der Erfindung liegt dabei in der Verwendung einer Suspension aus auf der Basis der in der DE 197 26 282 angegebenen Lehre ausgebildeten nanoskaligen Teilchen zur Herstellung eines medizinischen Präparats zur Behandlung von Arthrose, Arthritis und anderen rheumatischen Gelenkerkrankungen, wobei die Teilchen aus einem eisenoxidhaltigen Kern zur Erzeugung von Wärme in einem magnetischen Wechselfeld und einer inneren Schale mit zur Bildung von kationischen Gruppen befähigten Gruppen sowie mindestens einer äußeren Schale mit neutralen oder anionischen Gruppen sowie an die innere Schale gebundenen, durch Wärmeeinwirkung aktivierten Radionukliden und zytotoxisch wirkenden Stoffen oder Zytostatika bestehen und im Gelenkspalt sowohl in Makrophagen und proliferierenden Zellen der Synovia als auch den Zellen der Synovialis anbinden und wirksam sind. Die nanoskaligen Teilchen können des weiteren auch einschichtig ausgebildet sein und nur aus dem Kern und der inneren Schale - jeweils in der oben angegebenen Ausbildung - bestehen.

Es wurde gefunden, daß trotz der mit zunehmendem Alter der Patienten abnehmenden Phagozytoseaktivität der Makrophagen in der Gelenkflüssigkeit die intrazelluläre Aufnahme der erfindungsgemäß ausgebildeten Teilchen in Makrophagen selbst bei pathologisch verändertem Makrophagen-Titer im Gelenkspalt gesteigert wird und durch Adhäsion der Teilchen an aktiv proliferierenden Zellen der Synovia der Entzündungsprozeß eingedämmt wird. Aufgrund dieser Wirkungen und der durch Anlegen eines magnetischen Wechselfeldes erzeugten Wärme wird die Wirksamkeit der Radionuklide gegenüber der Radiosynoviorthese erhöht. Schließlich wird durch die Anlagerung von unter Wärmeeinfluß zytotoxisch wirkenden Stoffen der Behandlungserfolg über den additiven Effekt der Einzelkomponenten hinaus, nämlich durch die Kombination von Radio-, Thermo- und Chemotherapie, weiter verbessert.

In Ausgestaltung der Erfindung sind die Radionuklide Betastrahler mit geringer Reichweite und einer Halbwertzeit von einigen Stunden bis Tagen, wie zum Beispiel Y-90, Rh-186 oder Er-169, die mit Chelatbildnern an die nanoskaligen Teilchen gebunden sind. Als thermosensitiv wirkende Stoffe werden bevorzugt Doxorubicin, Epirubicin oder I-fostamid eingesetzt.

In weiterer Ausbildung der Erfindung liegt die Anregungsfrequenz des magnetischen Wechselfeldes zur Aktivierung des injizierten Präparats in Abhängigkeit von dessen Lokalisierung im Körper nach einem vorgegebenen Zeitablauf zwischen 1 kHz und 100 MHz.

In Ausgestaltung der Erfindung liegt die Eisenoxidkonzentration zwischen 0,01 und 50 mg/ml Synovialflüssigkeit, wobei die Leistungsabsorption zwischen 50 und 500 mW/mg Eisen und die Erwärmung zwischen 42 und 50°C liegt.

Die Nanoteilchen sind im Gelenkspalt durch Anlegen von statischen Magnetfeldgradienten lokal konzentrierbar. Nach Abklingen der Radioaktivität kann eine Mehrfachtherapie zur Behandlung von rezidivierenden Entzündungen durchgeführt werden.

Gemäß einem Ausführungsbeispiel zur Anwendung der Erfindung wird einer Suspension aus durch einen Eisenoxidkern und zwei Schalen gebildeten nanoskaligen Teilchen mit an diese gekoppeltem Doxorubicin als wärmesensitivem zytotoxischem Material und Betastrahlern als Radionuklide direkt in den zu behandelnden Gelenkspalt injiziert. Je nach Phagozytoseaktivität in der Synovia verbleibt die Suspension dort zunächst ohne Wärmeeinwirkung eine bestimmte Zeit, die prätherapeutisch festgelegt wird. Dieser Zeitraum kann zwischen einer Stunde und 72 Stunden liegen. In dieser Zeit werden die hier erfindungsgemäß zweischalig ausgebildeten Nanoteilchen von der Synovialflüssigkeit aufgenommen und wandern in die entzündete Synovialis ein. Mittels Magnetresonanztomographie wird zunächst kontrolliert, ob die Nanoteilchen tatsächlich in der Synovialis und in den benachbarten Lymphknoten und im Normalgewebe eingelagert sind. Gegebenenfalls wird eine Extravasation in benachbarte Bereiche vorgenommen, wovon jedoch aufgrund der hohen Phagozytoserate nicht auszugehen ist. Anschließend wird der betreffende Bereich einem magnetischen Wechselfeld mit einer Anregungsfrequenz zwischen 1 kHz und 100 MHz ausgesetzt, deren Höhe sich nach der Lokalisation der Erkrankung richtet. Während Hände und Arme mit höheren Frequenzen behandelt werden, sind bei Rückenbeschwerden oder tiefer gelegenen Gelenken oder Gelenken der Oberschenkel Frequenzen bis 500 kHz ausreichend.

Durch das Anlegen des magnetischen Wechselfeldes wird in dem betreffenden Gelenkbereich die örtlich begrenzte Wärme wirksam und gleichzeitig werden das Radionuklid und die zytotoxischen Stoffe, in diesem Fall das Doxorubicin, aktiviert, um mit dieser Dreifachtherapie und aufgrund der hohen Phagozytoserate und der differenziellen Endozytose der Nanoteilchen einen über der Addition der jeweiligen Einzelwirkungen liegenden Therapieerfolg zu erreichen. Das heißt, daß gegenüber den bekannten medizinischen Präparaten und Methoden zur Behandlung von rheumatischen Erkrankungen eine erheblich gesteigerte und bleibende Verödung (Fibrosierung/Sklerosierung) der Synovialis erfolgt.

Die aufgrund des auf die nanoskaligen Teilchen wirkenden magnetischen Wechselfeldes erzielbare Wärme bzw. die Dauer der Zuschaltung des magnetischen Wechselfeldes zur Erzielung einer bestimmten Gleichgewichtstemperatur wird anhand der Eisenoxid-Konzentration, die zwischen 0,01 und 50 mg/ml Synovialflüssigkeit liegt, und der typischerweise zwischen 50 und 500 mW/mg Eisen liegenden Leistungsabsorption im voraus berechnet. Die Feldstärke wird danach reduziert, um die Temperatur auf dem vorbestimmten Niveau von beispielsweise 45°C zu halten. Zwischen der behandelten Synovialschicht und dem benachbarten Knorpel und Knochen ist jedoch ein erheblicher Temperaturabfall zu verzeichnen, so daß bei der Wärmebehandlung die Knorpelschicht und der Knochen nicht geschädigt werden. Andererseits ist die Temperatur in der Knorpelschicht mit etwa 38 bis 40°C gegenüber normalen physiologischen Bedingungen leicht erhöht. Die dadurch bedingte Stimulation der Osteoblasten führt zu einer verbesserten Rekonstitution von bereits degenerativ veränderten Knochenrändern und Knorpel. Wiederholte Anwendungen des magnetischen Wechselfeldes wirken auch nach Abklingen der Radioaktivität nicht nur einer rezidivierenden Entzündung entgegen, sondern werden - bei einer Gleichgewichtstemperatur zwischen 38 und 41°C - auch zur Stimulation der Osteoblasten-Teilungsaktivität eingesetzt. Durch Anlegen von statischen Magnetfeldgradienten kann eine lokale Konzentration ("magnetisches Targeting") der Teilchen in dem behandelten Gelenk erfolgen.

## Patentansprüche

1. Verwendung einer Suspension aus nanoskaligen Teilchen, bestehend aus einem eisenoxidhaltigen Kern zur Erzeugung von Wärme in einem magnetischen Wechselfeld und einer inneren Schale mit zur Bildung von kationischen Gruppen befähigten Gruppen sowie wahlweise mindestens einer äußeren Schale mit neutralen oder anionischen Gruppen sowie an die innere Schale gebundenen, durch Wärmeeinwirkung aktivierten Radionukliden und zytotoxisch wirkenden Stoffen oder Zytostatika zur Herstellung eines medizinischen Präparats zur Behandlung von Arthrose, Arthritis und anderen rheumatischen Gelenkerkrankungen, wobei die in den Gelenkspalt eingebrachten Teilchen sowohl in Makrophagen und proliferierenden Zellen der Synovia als auch den Zellen der Synovialis anbinden und wirksam sind.

2. Verwendung nach Anspruch 1, wobei die Radionuklide zum Schutz von Knochen und Knorpel Betastrahler mit geringer Reichweite und einer Halbwertzeit von einigen Stunden bis Tagen sind.

3. Verwendung nach Anspruch 2, wobei die radioaktiven Betastrahler vorzugsweise Y-90, Rh-186 oder Er-169 sind.

4. Verwendung nach einem der Ansprüche 1 bis 3, wobei die Radionuklide mit Chelatbildnern an die nanoskaligen Teilchen gebunden sind.

5. Verwendung nach Anspruch 1, wobei die thermosensitiv wirkenden Stoffe vorzugsweise Doxorubicin, Epirubicin oder Ifosfamid sind.

6. Verwendung nach einem der Ansprüche 1 bis 5, wobei die Anregungsfrequenz des magnetischen Wechselfeldes zur Aktivierung des injizierten Präparats in Abhängigkeit von dessen Lokalisierung im Körper nach einem vorgegebenen Zeitablauf zwischen 1 kHz und 100 MHz liegt.

7. Verwendung nach einem der Ansprüche 1 bis 6, wobei die Eisenoxid-Konzentration zwischen 0,01 und 50 mg/ml Synovialflüssigkeit bei einer Leistungsabsorption zwischen 50 und 500 mW/mg Eisen und einer Erwärmung zwischen 42 und 50°C liegt.

8. Verwendung nach Anspruch 7, wobei die durch das Anlegen des magnetischen Wechselfeldes in der an die Synovialis angrenzenden Knorpel- und Knochenschicht erzielbare Temperatur zwischen 38 und 40°C liegt, wobei eine Stimulation der Osteoblasten erfolgt.

9. Verwendung nach einem der Ansprüche 1 bis 8, wobei die in den Gelenkspalt eingebrachten Nanoteilchen durch Anlegen von statischen Magnetfeldgradienten lokal konzentrierbar sind.

10. Verwendung nach einem der Ansprüche 1 bis 9, wobei eine Mehrfachtherapie nach Abklingen der Radioaktivität der Radionuklide zur Behandlung von rezidivierenden Entzündungen durchführbar ist.

## Claims

1. Use of a suspension of nanoscalar particles comprising a core containing iron oxide for generating heat in an alternating magnetic field and an inner shell with groups which are capable of forming cationic groups and optionally at least one outer shell with neutral or anionic groups and radionuclides activated by the influence of heat as well as cytotoxic agents and cytostatic drugs bound to the inner shell for the manufacture of a medical formulation for the treatment of arthrosis, arthritis and other rheumatic joint diseases, wherein the particles introduced into the joint are active in macrophages and in proliferating cells of the synovia as well as the cells of the synovialis.

2. Use according to claim 1, wherein the radionuclides are beta emitters of short range and a half-life in the range from several hours to several days to protect the cartilage and bones.

3. Use according to claim 2, wherein the radioactive beta emitters preferably are Y-90, Rh-186 or Er-169.

4. Use according to any one of claims 1 to 3, wherein the radionuclides are bound to the nanoscalar particles using chelating agents.

5. Use according to claim 1, wherein the thermosensitively acting agents preferably are doxorubicin, epirubicin or ifosfamide.

6. Use according to any one of claims 1 to 5, wherein the excitation frequency of the alternating electromagnetic field for activating the injected preparation after a predefined period of time is in the range from 1 kHz to 100 MHz depending on the location of the preparation in the body.

7. Use according to any one of claims 1 to 6, wherein the iron oxide concentration is in the range from 0.01 to 50 mg/ml of synovial fluid at a power absorption in the range from 50 to 500 mW/mg of iron and heating to a temperature in the range from 42 to 50°C.

8. Use according to claim 7, wherein the temperature that can be achieved in the cartilage and bone layer adjacent to the synovial membrane by applying the alternating magnetic field is between 38 and 40°C, which stimulates the osteoblasts.

9. Use according to any one of claims 1 to 8, wherein the nanoparticles are adapted to be concentrated by applying static magnetic field gradients, when introduced into the joint.

10. Use according to any one of claims 1 to 9, wherein a multiple therapy is applicable for the treatment of recurrent inflammations after decay of the radioactivity of the radionuclides.

## Revendications

1. Utilisation d'une suspension de particules nanométriques, consistant en un noyau contenant de l'oxyde de fer pour la génération de chaleur dans un champ magnétique alternatif, et en une enveloppe interne avec des groupes aptes à former des groupes cationiques, ainsi que, en option, en au moins une enveloppe extérieure avec des groupes neutres ou anioniques ainsi qu'avec des radionucléides liés à l'enveloppe intérieure et des substances à action cytotoxique ou des cytostatiques, activables par action de la chaleur, pour la fabrication d'une préparation médicale pour le traitement de l'arthrose, de l'arthrite et d'autres affections articulaires rhumatismales, les particules introduites dans l'interstice de l'articulation s'attachant aussi bien à des macrophages et à des cellules proliférantes de la synovie qu'à des cellules de la synoviale et étant actives.

2. Utilisation selon la revendication 1, pour la protection de l'os et du cartilage, les radionucléides étant des émetteurs de rayons bêta à faible portée et d'une période de quelques heures à quelques jours.

3. Utilisation selon la revendication 2, les émetteurs de rayons bêta étant de préférence Y-90, Rh-186 ou Er-169.

4. Utilisation selon l'une des revendications 1 à 3, les radionucléides étant attachés aux particules nanométriques par des chélatants.

5. Utilisation selon la revendication 1, les substances à effet thermosensible étant de préférence la doxorubicine, l'épirubicine ou l'ifosfamide.

6. Utilisation selon l'une des revendications 1 à 5, la fréquence d'excitation du champ magnétique alternatif pour l'activation de la préparation injectée étant comprise entre 1 kHz et 100 MHz d'après un déroulement chronologique prédéfini en fonction de sa localisation dans le corps.

7. Utilisation selon l'une des revendications 1 à 6, la concentration en oxyde de fer étant comprise entre 0,01 et 50 mg/ml de liquide synovial pour une absorption d'énergie entre 50 et 500 mW/mg de fer et un échauffement entre 42 et 50 °C.

8. Utilisation selon la revendication 7, la température réalisable par l'application du champ magnétique alternatif dans la couche de cartilage et d'os contiguë à la synoviale étant comprise entre 38 et 40 °C, moyennant quoi s'effectue une stimulation des ostéoblastes.

9. Utilisation selon l'une des revendications 1 à 8, les nanoparticules introduites dans l'interstice articulaire étant susceptibles d'être concentrées localement par l'application de gradients statiques de champ magnétique.

10. Utilisation selon l'une des revendications 1 à 9, une thérapie multiple étant réalisable pour le traitement d'inflammations récidivantes après la baisse de la radioactivité des radionucléides.
